Europäisches Patentamt

European Patent Office

Office européen des brevets

(19)

(11) **EP 1 482 037 A1**

(12) # EUROPEAN PATENT APPLICATION

(43) Date of publication:
**01.12.2004 Bulletin 2004/49**

(51) Int Cl.⁷: **C12N 15/11**, A61K 48/00, C07H 21/02, C07H 21/04

(21) Application number: **04253066.7**

(22) Date of filing: **25.05.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **30.05.2003 US 475360 P**

(71) Applicant: **Yeung, Wah Hin Alex**
**Hong Kong (CN)**

(72) Inventor: **Yeung, Wah Hin Alex**
**Hong Kong (CN)**

(74) Representative: **Evans, Claire**
**Marks & Clerk**
**Incorporating Edward Evans Barker**
**Clifford's Inn**
**Fetter Lane**
**London EC4A 1BZ (GB)**

(54) **Inhibition of gene expression by delivery of specially selected double stranded or other forms of small interfering RNA precursors enabling the formation of small interfering RNA in vivo and in vitro**

(57) The use of specially selected sequences from the target gene into designing double stranded or other forms of RNA (siRNA precursors or siRNAp) that enables small interfering RNA (siRNA) from this new invention is delivered for inhibition of cellular gene expression. Diseases may be prevented and treated by this process, e.g. Severe Acute Respiratory Syndrome (SARS) and Human Immunodeficiency Virus (HIV) infections. The process may be practiced in vivo or in vitro. The small interfering RNA enabled is of sequences usually of 23 nucleotides or less. The invented method of sequence selection from the target gene, however, maybe applicable to double stranded RNA of any length.

EP 1 482 037 A1

Description

## BACKGROUND OF THE INVENTION

### FIELD OF THE INVENTION:

The present invention relates to the gene specific inhibition by double stranded ribonucleic acid (dsRNA) that enables small interfering RNA (siRNA). The selection of the number and sequences of the dsRNA created depends on the criteria set forth by this invention. In addition, the special delivery method of the selected dsRNA enabling siRNA will try to solve the difficulties in preventing and treating SARS and HIV infections.

### GENERAL DESCRIPTION OF THE RELATED ART:

In the recent years, RNA interference creates much excitement in the biological field. The first sign of such an interference was shown in 1990 when biologist Rich Jorgensen tried to turn purple petunias more purple. He inserted a second copy of the rate-limiting enzyme gene. But instead of purple, the petunia was found to be white. He called this paradoxical effect "co-suppression" but at that time nobody knew why adding more of a gene that promoted a special color turned that gene off instead. Five years later, an experiment using a single strand of control "sense" strand of RNA worked as well to suppress the intended gene than the "anti-sense" strand. In 1998 Andrew Fire and Craig Mello solved the mystery by demonstrating that double stranded RNA was the real silencing agent. They called this "RNA interference" and a new field was born (*Blocker B. RNA interference dazzles research community. Journal of the National Cancer Institute: Vol. 95, No. 7, April 2, 2003*). The Tuschl group extended the technique to mammalian cells and has made RNA interference or RNAi what it is today (*Elbashir S.M., Harborth J., Weber K., Tuschl T. Analysis of gene function in somatic mammalian cells using small interfering RNAs. Academic Press: Methods 26 (2002): 199-213*).

In mammals, double stranded RNA or dsRNA acts mainly through post transcriptional mechanism targeting mRNA for destruction and the mediators for this sequence specific target recognition is now known to be consisted of about 21 nucleotide small interfering RNA (siRNA). These small siRNAs are produced normally from a much longer dsRNA that occurs in a natural state by a reaction involving Dicer Rnase III. After these siRNAs are formed, they are again taken up by another ribonuclease protein called RNA-inducing silencing complex (RISC). The RISC protein ultimately unwinds the siRNA to form a single strand and this will guide the RISC complex towards cytoplasmic target mRNA degradation (*Hannon G.J. RNA interference. Nature: Vol. 418, July 11, 2002*). In certain species, siRNA RISC complexes may also be able to incorporate into sequence specific DNA through chromatin or other sites that can effectively change genetic expression of that gene. Transitive RNAi can also occur if these siRNA are complementary to other RNA of the same or different targets. In some organisms, RNA-dependent or directed RNA polymerase (RdRP) can also prime siRNA synthesis using the target mRNA as template. The target RNA is then inactivated by Dicer RNA cleavage rather than by RISC. Some of the effects of siRNA silencing the target mRNA may sometimes be able also to amplify and spread throughout the organism, even when triggered by only minute quantities of dsRNA. This effect, however, has not been observed in mammals so far.

SiRNA duplexes, deriving from double stranded or other precursors, are now widely used for silencing of mammalian genes in cultured cells. The introduction and formation of these functional siRNAs are now done by a variety of methods including transfection, electroporation, plasmid based and viral based expression systems.

Delivery of siRNAs directly into animals have so far be limited to only a few examples. Recombinant adenoviruses expressing siRNAs under the control of a CMV promotor reduced target gene expression in mouse liver or brain, when injected into the tail vein or the brain striatal region of the animals. The only real disease prevention so far was performed by Lieberman in which they gave mice three massive high pressure injections equivalent to about half the animals' blood volume of a solution that forced the siRNA targeting the Fas gene into the liver (*Couzin J. RNAi to the liver's rescue: ScienceNOW- Couzin 2003 (210): 1*). About 80% to 90% of liver cells incorporated the RNA molecules. The next day, the animals got an antibody that send Fas into overdrive, causing liver failure. Most of the control mice died while 82% of the treated mice lived.

The success of this first animal study would surely lead to future research to use this technology for human diseases.

### SUMMARY OF THE INVENTION:

As described above, the RISC siRNA complex has not been clearly defined. The task of selecting the right sequences to be used as the starting dsRNA or siRNAp template has not been standardized. In practice, there may be hundreds of combinations of sequences that may fit the criteria of the 19-23 nucleotides (nt) dsRNA or siRNAp. To make a decision to pick the right one is therefore difficult at best, if not impossible. This invention is to define the sequences in the target gene to look for when trying to construct a better siRNAp or dsRNA.

Delivery of siRNA to certain diseases that target white blood cell in general or lymphocyte in particular is also difficult. In this invention, delivery of these specially designed siRNAs to the respiratory system and in vitro stem cells directly can circumvent some of the difficulties in treating SARS and HIV virus infections.

**DETAILED DESCRIPTION OF THE INVENTION:**

METHOD OF SELECTING THE RIGHT SEQUENCES FOR dsRNA AND siRNAp

Candidate target regions are selected from gene sequences on the basis of several well-known criteria. We search at least 50bp downstream from the transcriptional start site and identify regions fitting the pattern AA(n19)TT, where n is equal to any base. However if this is not possible, other less perfect sequences are selected (eg. nA(n19) Tn, nA(n19)nn). They must have approximately 50% GC ratio and be devoid of long series of nucleotide repeats. This selection yields a 21-nt dsRNA oligo, but this technique can be used to generate oligos of any length.

These siRNA candidates are then screened for downstream or upstream homologous regions that may generate natural hairpins or other secondary mRNA foldings. The ten bases at the 5' end and the ten at the 3' end of the candidate region, either including (table 1) or excluding (table 2) the first and last two bases, are read in the 3'-5' direction and any compliments of these regions located on the same mRNA are identified. Original candidate regions are scored on the basis of the number of downstream base-pair matches, and those with the most complement sequence homology are selected (tables 1-3).

Complimentary dsRNA oligos are generated so the antisense strand is complimentary to this 19bp central region. Each strand has a 3' dTdT overhang, of which both, or only the 5' dT of which, is complimentary to the corresponding 5' region of the target. Candidate sequences are screened and matched, working against the relevant BLAST database (http://www.ncbi.nlm.nih.gov/BLAST/) to check systematically for non-specific homology and those with over 75% homology are excluded. siRNA oligos are ordered and synthesized by a commercial oligonucleotide synthesis company (Proligo) or other delivery mechanisms employed.

| *Start nt* | *23nt target sequence, 5'–3'* | *5'Homologues'* | *3' Homologues* | *Homologous matches* | **Human Blast cross-matches** |
|---|---|---|---|---|---|
| **245** | CACCGTTCATTCTAGA GCAAACA | 106 - GGTG | 191- TGTTTG | 10 | *16/16 (23/23)* |
| **142** | AACCCTAACTGAGAA GGGCGTAG | 95- AGGGTT | No homologues | 6 | *17/17* |
| *265* | AAAAAATGTCAGCTGC TGGCCCG | 130- CATTTTTT | 213 - CGGGC | 13 | *17/17* |

Table 1. Potential siRNA that targets the 23-nt regions of the human telomerase RNA (hTR). Final regions are based on the 19-nt recognition sequence in addition to the 3' dTdT overhang- matching bases. Final siRNA selection is based on the number of homologous matches in addition to other criteria. Downstream compliment homolgues are numbered from their base count origin.

| Base count origin | 19nt Sequence, 5'–3' | 5' homologues | 3' homologues | Homologous matches | Human Blast cross-matches |
|---|---|---|---|---|---|
| 1953 | TTGTGAACATGGACTACGT | 3060 - ACAA | 2780 - GTAG | 8 | 15/15 |
| 1741 | TGTCACGGAGACCACGTTT | 2672 - GTGACA | 1984 – AACGT | 11 | 14/14 |
| 2241 | TCGCCAGCATCATCAAACC | 2594 - GGCGA 3912 - GGCGA | 3087 – GGTTT | 10 | 19/19 |

Table 2. Potential siRNA-tragetting regions of the human telomerase reverse transcriptase subunit gene (hTERT). Regions are selected solely on the basis of the 19nt internal identity sequence. Final siRNA selection is based on the number of homologous matches in addition to other criteria. Complimentary homolgues are numbered from their base count origin.

| Base count origin | 21nt target sequence, 5'–3' | 5' homologues | 3' homologues | Homologous matches | Human Blast cross-matches |
|---|---|---|---|---|---|
| 1937 | AAGAGCAGCTGTCACCATACT | 2615 - TGCTCTT | 108 - AGTAT | 12 | 16/16 |
| 2555 | ATGGCCTCATGCTCTTAGAGA | 1584 - AGGCCAT | 45 - TCTCTAA | 14 | 15/15 |
| 3409 | ACAAGGCAACCAATGGTGCCA | 257 - GCCTTGT | 393 - TGGCAC | 13 | 15/15 |

Table 3. Potential siRNA-tragetting regions of the SARS replicase (pol)-coding region. Regions are selected on the basis of the 21bp internal homologous sequence (one base from each overhang). Final siRNA selection is based on the number of homologous matches in addition to other criteria. Compliment homolgues are numbered from their base count origin.

## COMPUTER ASSISTED SEQUENCE SELECTION

siRNA-targeting regions are quickly identified using computer software programmed to recognize these specific, simple criteria. Currently available software such as Primer Express (Applied Biosystems), BLAST (National Centre for Biotechnology Information), Gene-Tool Lite (BioTools Inc.) perform very similar functions but we have yet to discover a software tool able to search for short downstream reverse-complimentary sequences. It is anticipated that such a software tool will be available in the near future.

Further regional selection, if more than one regions have similar matching sequences and RNA preference index (RPI) defined in the working formula section that follows, can be assisted by sequence comparison and alignment algorithms known in the art (see Gribskov and Devereux, Sequence Analysis Primer, Stockton Press, 1991) and calculating the likely binding resulting from different degrees of homology, using various algorithms and software packages (eg. GeneTool lite).

## THE WORKING FORMULA BEHIND THIS INVENTION

The present invention is to improve both the efficacy and the specificity of the resulting siRNA, plus other yet to be determined advantages. The exact nature of RNA interference and silencing has not been precisely developed in any organism, even less in mammals. The structures and functions of the RISC protein and the particular strand of sense or non-sense RNA be used are still being investigated. The present invention is to select the best possible combination of sequences out of hundreds or thousands of combinations available in a given target.

The selection of a downstream or upstream complimentary region or regions that ideally be 5' to 3' or 3' to 5' (and it may also be 5' to 5', 3' to 3'), matching as many nucleotide bases as possible and then the rest to be selected by standard comparison and alignment algorithms will achieve multiple theoretically important purposes.

The first idea is to select as many complimentary nucleotide bases as possible. It would be ideal to have all 19nt matched. If that is not possible, which is most likely, then the selection will go to the one that have the most nucleotide bases matched. For example, selection for a set of 19 nt dsRNA (it could be of other nucleotide length) will go for a set that has the highest number of nucleotides matched. A set that have eight of the nucleotide matched will be theoretically better than the one with only six or seven. The matched nucleotides should start from the first or the third nucleotide of the strand although this is not an absolute prerequisite. Alternatively, there may be more than one matching segment in each region, e.g. match may be achieved by 1-12, or 2-7 and 9-14 and so on.

The second idea is to select as many such complimentary regions for the same set of nucleotides as possible, if it exists. For example, one would find, that there are three sets of 19 nt dsRNA and all of them have 6 nts matched. One such set has an extra 6 nt region matched further downstream or upstream, and the other two are restricted to one 6 nt region. Then the logic is to select that first one with more than 1 of the 6 nt region matched. The idea is always to go for the set that has more than 1 region matched. The more regions are the better.

Sometimes it may be difficult to select between two sets of nucleotide with one having more bases matched and the other with more regions matched. At the present time we would prefer choosing the one with at least a four to five nucleotide match AND with the most number of matched regions. The reason behind this is probably the RISC protein is likely to operate an unwound segment of at least four to five nucleotide single strand RNA to guide its silencing mode. In this case, the more regions of match will offer more chances of a silencing. It may, however, turn out later that the RISC protein operates with a longer strand of RNA as a guide, then more nucleotide matched will be the preferred overwhelming selection, rather than the more regions matched. In any case, the decision to use this invention is to focus on how siRNA functions as more of its mysteries begin to unfold.

A simple working formula to follow:

1. The first preference is to seek the highest sequence complementary index (Sc) defined as

$$Sc = M/Risc$$

Whereas Sc is the Sequence complementary index

M is the total number of nucleotides of matched sequence segment or segments that exceed the minimum requirement length of sequence operated as the guide single strand RNA for the RISC protein. For example, if a chosen region has 3 segments that matched, two exceeding five consecutive nucleotides like 1-6, 8-15 and 1 segment has only four consecutive nucleotides like 17-20 and the minimum required strand length of the RISC protein is 5, then only the first 2 segments will qualify for calculation purposes.

$$M = (1-6) + (8-15)$$

$$M = 6+8$$

$$M = 14$$

Risc is the minimum strength length of nucleotide that operates in that particular system. It may change in different species or in different systems, e.g. in RdRP. In the above case it is set as 5.

$$Sc = 14/5$$

$$Sc = 2.8$$

2. The second criteria is to choose the number of regions that have at least one segment of sequences that exceeds the minimal operating single strand length of the RISC protein.

N = Number of regions that have at least one segment matched exceeding Risc requirement

3. The final conclusion of this working formula is

$$RPI = Sc \times N$$

RPI is the RNA preference index for the selection of the dsRNA or siRNAp used, the higher the index, the more preferred is the selection

4. If different RNA sets have the same or very similar RPI, the selection will then go to the one that has the best comparison and alignment algorithms.

It would be apparent to those skilled in the art that the above formula is one of many ways to make use of this invention. The important criteria of selecting more specific targets and more complementary regions, balanced against the working confines of the siRNA system chosen, will enable those skilled in the art to use this invention to its fullest extent.

SUMMARY OF THE THEORIES BEHIND THE INVENTION

The advantages of the selection based upon this invention are multiple. Since RISC protein will eventually unwind the dsRNA into a single strand with possible modification of the number of nucleotides carried on such strand, it would be vital to have the specific nucleotides that are guiding the RISC protein to the target gene be exposed to as many targets as possible. If there are more than one of these matching nucleotides regions on the target genes, then the chance of such silencing will be increased among a host of other factors intra-cellularly that may obscure such a process. On the other hand, the sense nucleotide strand of the RISC protein, which normally serves no function, is now able to complement the selected matched region. The efficacy of the specially selected siRNA is thus at least two times more than the randomly selection method being used before. A speculative theory put forwards by this invention, besides the fact that the efficacy of siRNA molecules and targets is increased two or more folds, is that the mammalian system is indeed a much more complicated system. More confirmation information is indeed required before silencing is specific. A single target region being brought about by one single segment of siRNA with the RISC protein may not be read as the ideal system. If, for example, the target RNA is being silenced simultaneously by two or more regions proposed by this invention, then such a targeted silencing signal is likely to be confirmed. This more specific confirmation system may be extremely important before RNA interference in mammalian cell can be made effective for a long time or be permanently passed on to other cell systemically. The ability to transfect or introduce siRNA that can inhibit mammalian cell genetically for a long period of time as well as rendering systemic cell acquired such a silencing effect is of the utmost importance for the prevention and treatment of disease. One interesting observation why this is so is the occurrence of naturally looped RNA in RNA silencing. This looped RNA, in the form of a hairpin, creates the dsRNA, cut off into smaller pieces by Dicer, and then reassembled by the RISC protein to become the single strand guided siRNA. There are suggested similarities in the structure and transference of the RNA strands between the dicer and RISC protein (*Hammond S.M., Boettcher S., Caudy A.A., Kobayashi R., Hannon G.J. Argonaute2, a link between genetic and biochemical analyses of RNAi. ScienceMAG - Hammond et al. 293 (5532): 1146*). Such a theory indicates that the system works by small strands that have to be specific. Since it is in the nature that for the loop to occur, there must be complimentary regions in the natural form of the target gene. And if the system is to be working in a "small strand" theory, the best confirmation is to be able to have two or more regions silenced to confirm to the affected cell that such a specific action should take place. If this confirmation is strong, then the message would then be permanent and be passed on to the other cell of the organism. Even though this is only a hypothesis, there are plenty of examples in nature that would validate such a theory. For examples, genes are made up of two sets of complimentary nucleotides only and not by many more nucleotides that are different. It is therefore very likely, that in order for nature to recognize that a definite important action like gene silencing has to take place, or to take place for a long time, or to take place within the whole organism, confirmation should be received through two or more (though shorter) sets of instruction simultaneously rather than by only one (though more specific and complicated) longer set. This invention is thus formulated from all of the above theories.

METHODS OF TRANSFECTION, ELECTROPORATION OR VECTOR EXPRESSION SYSTEM

The various methods of inducing siRNA-mediated gene-silencing include, but are not limited to, introduction of naked, double stranded siRNA; introduction of single-stranded RNA so produced to form a double-stranded hairpin structure; introduction of a DNA plasmid containing the appropriate promoter sequences to induce synthesis of 2 complimentary strands of RNA which will hybridise at the site of transcription or elsewhere giving rise to a double-stranded RNA entity; introduction of a recombinant virus so constructed so as to transcribe 2 complimentary strands of RNA which hybridise following transcription giving rise to a double-stranded RNA entity; introduction of recombinant bacteria containing genetic elements enabling production of complimentary double-stranded RNA entities at the site of transcription or elsewhere and any of the above systems whereby a single-stranded RNA is produced of appropriate sequence to form a double-stranded hairpin or other double-stranded structure upon transcription.

Cell-lines or other cells (stem cells, lymphocytes, etc) are transfected in vitro with siRNA selected according to the criteria set out above. Cells are useful for a variety of purposes detailed in US Patent Applications 20020114784, 20020173478, 20030056235 and 20020132788.

For transfection of cell-lines, a total of 60pM of siRNA (3μl of a 20μM solution) is added to each well of a 24-well plate grown to 30-50% confluence. For each well, siRNA is added to 50μl of Opti-MEM Reduced Serum Medium (Invitrogen) and mixed gently. In a separate tube, 3μl of Oligofectamine transfection reagent (Invitrogen) is mixed with 12μl Opti-MEM and mixed gently before incubation for 5 minutes at room temperature. The contents of the tubes is combined and incubated for 20 minutes at room temperature to allow siRNA-Oligofectamine complexes to form. 68μl is added to each well. Plates are incubated at 37°C for various times depending on their final use. If further transfection is necessary, cells can be passaged and re-transfected after 72 hours or more.

METHODS OF DELIVERY TO SPECIAL DISEASES

Methods of delivery of this specially designed dsRNA or siRNAp include all forms commonly known to those practicing in the art.

RNA may be directly introduced into the cell, intracellularly. It may also be delivered into a cavity belonging to the organism, such as the respiratory system, pleural cavity or interstitial space. It may also be introduced into the circulation of the organism by different forms of injections such as intravenously, intra-arterially, or intramuscularly. Vascular or extravascular circulation, the blood or lymph system, the roots, and the cerebrospinal fluid are all sites whereby this specially designed RNA may be introduced. It may be introduced orally or be introduced by bathing an organism directly. It may be sprayed onto a plant. It may be expressed by genetically modification of the primary or targeted organism or by introducing another organism or vehicle that has been genetically modified to express this RNA into the primary or targeted organism. A transgenic organism that expresses this specially designed RNA from a recombinant construct may also be produced by introducing the construct into a zygote, an embryonic stem cell, or another multipotent cell derived from the appropriate organism.

Physical methods of introducing this specially designed RNA include injection of a solution containing the RNA, or bombardment by small particles covered or containing the RNA, soaking the cell or organism in a solution of the RNA, or electroporation of cell membranes in the presence of the RNA. Other common methods known to the arts may also include the use of lipid-mediated carrier transport and chemical mediated transport system. A viral construct can also be packaged into a viral particle and when introduced into the cell of an organism, accomplish efficient introduction of the expression conduct leading to the specially designed RNA. This specially designed RNA may also be introduced along with components that could enhance the uptake of RNA by the cell, promote annealing of the duplex strands, stabilize the strands and increase inhibition of the target gene.

This invention also includes a delivery method that would serve as a vaccine for pulmonary infection such as from the SARS virus or other infections. The RNA is introduced to the upper and lower respiratory system of a human being by commonly used techniques such as a nebulizer. Incorporation of the genetic inhibition from this RNA into the cells of the respiratory system and the immune cells present in that system will enable the prevention of a specific infectious disease, thereby a vaccine is created.

This invention also includes a delivery method that would introduce the RNA into a stem cell or immune function cell ex vivo. The cell, after incorporation of the RNA for genetic inhibition, is introduced back to the organism or human being for prevention and treatment of disease. The cell, although be likely to expand and differentiate within the organism, is by no means able to effect changes in the genetic expression of that organism. Treatment of the HIV infection is one prime example how the above delivery method can be used.

FIELDS OF USAGE

The present invention and the special RNA selected for genetic inhibition may be used for the treatment and prevention of disease. One example is to use this RNA and be introduced into a cancer cell or tumor and thereby inhibits genetic expression of a gene required for the initiation or maintenance of the cancer phenotype. This can also be applied for the prevention of cancer by selecting inhibit the oncogene or genes necessary for the transformation of a benign phenotype into a cancerous phenotype. This treatment could be used in almost all types of cancer, by itself, or be used in combination with other treatment modalities such as chemotherapy, radiation therapy and surgery etc.

The present invention also can target a gene derived from any pathogen. For example, the gene could cause immunosuppression of the host directly or be vital for the replication of the pathogen, transmission and expansion of the infection. The RNA can then be introduced into potentially affected cells, such as the immune cells or stem cell ex vivo and then reintroduced back to the host to prevent or treat the pathogen. Alternately, the RNA can be delivered by different methods to the cells at risk, for example, by a nebulizer to the respiratory system of a SARS patient. The inhibition of the SARS viral genome by the still viable respiratory cells will be able to prevent further spreading of the virus, thus completing the treatment objective.

The present invention is not limited to any type of target gene or nucleotide sequence.

The following classes of possible target genes are listed for illustrative purposes: developmental genes, oncogenes, tumor suppressor genes, enzyme genes etc.

The present invention could also be applied as a methodology to produce plants with less susceptibility to climate injury, insect infestation, pathogen infection, and ripening characteristics. In fact, any gene or genes that may be useful in the agricultural community could be a potential target or targets of such specially selected RNAs.

The present invention could also be used to identify gene function in an organism comprising the use of the RNA to inhibit the activity of a target gene of previously unknown function. In reverse, it could also be used to study the effect of a knock down of certain known function gene on that organism. The invention could be used in determining potential targets for pharmaceutical development and for determining signaling pathways responsible for development and aging, etc. It is foreseeable that the invention can be used to develop prevention and treatment methods of a variety of diseases apart from infection and cancer.

The present invention could also be used in a variety of diagnostic methods and gene mapping studies. It could be used in high throughput screening. It could be used to as a component of a kit. Such a kit may also include instructions to allow a user of the kit to practice the invention.

While the present invention has been described with methods and embodiments that are considered to be standard and practical, it is understood that the invention is not to be limited or restricted to the disclosed ones. On the contrary, it is intended to cover various modifications and similar arrangements and methodologies within the spirit and scope of the appended claims.

It is the intention that variations in the described invention will be obvious to those skilled in the art without departing from the novel aspects of the invention and such variations are intended to come within the scope of the present invention.

**Claims**

1. A process to inhibit expression of a target gene in a cell by introducing a double stranded or other forms of RNA (dsRNA and other siRNA precursors "siRNAp") enabling small interfering ribonucleic acid (siRNA), wherein the dsRNA and siRNA precursors (siRNAp) comprise, in most cases, a double stranded structure or a single stranded hairpin structure with an identical or near identical sequence as compared to a region of the target gene. The region selected for inhibition, by this invention, will be a region that should have another complementary or near complementary region or regions in the same target gene so that a natural hairpin structure of a double stranded ribonucleic acid (dsRNA) or multiples of the same or different hairpin dsRNA could have been formed, at least in theory, de novo from that particular targeted gene. For practical purposes, the best match sometimes can only be found for the first 4-12 nucleotides or so since the chance of a match is exponentially more difficult after that number. The match may not be restricted to start from the first 4-12 nucleotides but may start and end from and in any position. This invention is to apply this concept for the selection of the right sequences for dsRNA and siRNAp to enable target gene silencing.

2. The method of claim 1, wherein the selected region to target has another complementary region or regions that can have a perfect match of the usual 19-23 nucleotides selected for the function of siRNA.

3. The method of claim 1, wherein the selected region to target has another complementary or regions that can have a match of any number less than the full 19-23 nucleotides selected for the function of siRNA.

4. The method of claim 1, wherein the selected region to target has another complementary region or regions that can have a match starting from the first nucleotide. For example, the match of 8 nucleotides starting from the first, i.e., the match is from 1-8.

5. The method of claim 1, wherein the selected region to target has another complementary region or regions that can have a match starting from any position. For example, the match can be from 2-9, or 3-11 and so on.

**6.** The method of claim 1, wherein the selected region to target has another complementary region or regions that can have a match of more than 1 segment within that same stretch of 19-23 nucleotides. For example, the match can be from 2-9 **and** 11-21, or 1-4, 6-10 **and** 13- 19 and so on.

**7.** The method of claim 1, wherein the selected region to target has another complementary region or regions measuring from either direction of 5' to 3' of the original selected region.

**8.** The method of claim 1, wherein the selected region to target has only one such complementary region in the same gene.

**9.** The methods of claim 1, wherein the selected region to target has more than one such complementary region in the same gene.

**10.** The method of claim 1, wherein the selected region may have complementary region or regions in more than one gene to achieve multiple target genes silencing.

**11.** The method of claim 1, wherein the dsRNA and siRNAp enabling siRNA is defined as having no more than 23 nucleotides.

**12.** The method of claim 1, wherein the dsRNA and siRNAp enabling RNAi have 24 or more nucleotides.

**13.** The method of claim 1, wherein the target gene is a cellular gene.

**14.** The method of claim 1, wherein the target gene is an endogenous gene.

**15.** The method of claim 1, wherein the target gene is a transgene.

**16.** The method of claim 1, wherein the target gene is a viral gene, from either a natural or an artificial source.

**17.** The method of claim 1, wherein the cell is from a human being.

**18.** The method of claim 1, wherein the cell is from any other animal.

**19.** The method of claim 1, wherein the cell is from a plant.

**20.** The method of claim 1, wherein the siRNAp or dsRNA comprise one strand which is self-complementary.

**21.** The method of claim 1, wherein the siRNAp or dsRNA comprise of two separate complementary strands.

**22.** The method of claim 1, wherein the delivery of the RNA is to a cell taken out from an organism and target inhibition is done ex vivo. The cell is then given back to the same or different organism to enable the intended use of the gene inhibition.

**23.** The method of claim 1, wherein the cell is present in a first organism, and the RNA is introduced to the first organism by the introduction of a second RNA containing organism to the first organism.

**24.** The method of claim 1, wherein an expression construct in the cell is introduced to engineer the production of the desired RNA duplex.

**25.** The method of claim 1, wherein the target cell is a tumor cell, benign or malignant, and the prevention and treatment of such in an organism.

**26.** The method of claim 1, wherein the final goal for the gene inhibition is for the prevention and treatment of infections by any pathogens.

**27.** The method of claim 1, wherein the final goal for the gene inhibition is for diagnostic purposes, for kit assembly and high throughput screening.

**28.** The method of claim 1, wherein the final goal for the gene inhibition is for the development of RNA-based drugs that disrupt the target cell expression at the mRNA level, so as to treat human or animal disease.

**29.** The method of claim 1, wherein the final goal for the gene inhibition is for the development of RNA based technology for the fight against biochemical warfare or terrorism, such as but not limited to inhalation prevention and treatment of anthrax etc.

**European Patent Office**

## PARTIAL EUROPEAN SEARCH REPORT

which under Rule 45 of the European Patent Convention shall be considered, for the purposes of subsequent proceedings, as the European search report

**Application Number**

EP 04 25 3066

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 03/006477 A (MCLACHLAN JUANITA ; MELLO CRAIG C (US); GRISHOK ALLA (US); HUTVAGNER G) 23 January 2003 (2003-01-23) <br> * sentence 7 - sentence 10 * <br> * sentence 51; figure 27; examples 3,6,7 * <br> ----- | 1-29 | C12N15/11 <br> A61K48/00 <br> C07H21/02 <br> C07H21/04 |
| Y | KAWASAKI H ET AL: "siRNAs generated by recombinant human dicer induce specific and significant but target site-independent gene silencing in human cells" <br> NUCLEIC ACIDS RESEARCH, OXFORD UNIVERSITY PRESS, SURREY, GB, <br> vol. 31, no. 3, <br> 1 February 2003 (2003-02-01), pages 981-987, XP002971836 <br> ISSN: 0305-1048 <br> * the whole document * <br> ----- | 1-29 | |
| A | US 2003/084471 A1 (BEACH DAVID ET AL) 1 May 2003 (2003-05-01) <br> * the whole document * <br> ----- <br> -/-- | 1-29 | **TECHNICAL FIELDS SEARCHED (Int.Cl.7)** <br> C12N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 28 July 2004 | Mossier, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

Although claims 1 - 29 encompass methods of treatment of the human/animal body (Article 52(4) EPC), the search has been carried out and based on the alleged effects of the compound/composition.

- - - - -

**EP 1 482 037 A1**

European Patent
Office

**PARTIAL EUROPEAN SEARCH REPORT**

Application Number

EP 04 25 3066

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim* | |
| A | MCMANUS M T ET AL: "GENE SILENCING USING MICRO-RNA DESIGNED HAIRPINS" RNA, CAMBRIDGE UNIVERSITY PRESS, CAMBRIDGE, GB, vol. 8, no. 6, June 2002 (2002-06), pages 842-850, XP008021481 ISSN: 1355-8382 * the whole document * | 1-29 | |
| A | PADDISON PATRICK J ET AL: "Short hairpin RNAs (shRNAs) induce sequence-specific silencing in mammalian cells" GENES AND DEVELOPMENT, COLD SPRING HARBOR LABORATORY PRESS, NEW YORK, US, vol. 16, no. 8, 15 April 2002 (2002-04-15), pages 948-958, XP002204653 ISSN: 0890-9369 * abstract * | | |
| A | KAWASAKI H ET AL: "Short hairpin type of dsRNAs that are controlled by tRNA Val promoter significantly induce RNAi-mediated gene silencing in the cytoplasm of human cells" NUCLEIC ACIDS RESEARCH, OXFORD UNIVERSITY PRESS, SURREY, GB, vol. 31, no. 2, January 2003 (2003-01), pages 700-707, XP002965487 ISSN: 0305-1048 * abstract * * page 705, column 1, paragraph 3 - page 706, column 1, paragraph 4; figure 1 * | 1-29 | TECHNICAL FIELDS SEARCHED (Int.Cl.7) |

-/--

EPO FORM 1503 03.82 (P04C10)

13

**European Patent Office**

**PARTIAL EUROPEAN SEARCH REPORT**

Application Number

EP 04 25 3066

## DOCUMENTS CONSIDERED TO BE RELEVANT

| Category | Citation of document with indication, where appropriate, of relevant passages | Relevant to claim |
|---|---|---|
| A | AGAMI R: "RNAi and related mechanisms and their potential use for therapy" CURRENT OPINION IN CHEMICAL BIOLOGY, CURRENT BIOLOGY LTD, LONDON, GB, vol. 6, 2002, pages 829-834, XP002239988 ISSN: 1367-5931 * the whole document * | 1-29 |
| A | CERUTTI H: "RNA interference: traveling in the cell and gaining functions?" TRENDS IN GENETICS, ELSEVIER, AMSTERDAM, NL, vol. 19, no. 1, January 2003 (2003-01), pages 39-46, XP004398856 ISSN: 0168-9525 * the whole document * | 1-29 |
| A | WO 02/055693 A (RIBOPHARMA AG ; ROST SYLVIA (DE); KREUTZER ROLAND (DE); LIMMER STEPHAN) 18 July 2002 (2002-07-18) * abstract * | 1-29 |

CLASSIFICATION OF THE APPLICATION (Int.Cl.7)

TECHNICAL FIELDS SEARCHED (Int.Cl.7)

EPO FORM 1503 03.82 (P04C10)

## ANNEX TO THE EUROPEAN SEARCH REPORT
## ON EUROPEAN PATENT APPLICATION NO.

EP 04 25 3066

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2004

| Patent document cited in search report | | Publication date | Patent family member(s) | | Publication date |
|---|---|---|---|---|---|
| WO 03006477 | A | 23-01-2003 | BR | 0211111 A | 22-06-2004 |
| | | | CA | 2453183 A1 | 23-01-2003 |
| | | | EP | 1412371 A1 | 28-04-2004 |
| | | | WO | 03006477 A1 | 23-01-2003 |
| US 2003084471 | A1 | 01-05-2003 | WO | 03062394 A2 | 31-07-2003 |
| | | | US | 2004086884 A1 | 06-05-2004 |
| | | | AU | 4579301 A | 24-09-2001 |
| | | | CA | 2403397 A1 | 20-09-2001 |
| | | | EP | 1272630 A2 | 08-01-2003 |
| | | | JP | 2003526367 T | 09-09-2003 |
| | | | US | 2004018999 A1 | 29-01-2004 |
| | | | US | 2002162126 A1 | 31-10-2002 |
| | | | WO | 0168836 A2 | 20-09-2001 |
| WO 02055693 | A | 18-07-2002 | DE | 10100586 C1 | 11-04-2002 |
| | | | DE | 10160151 A1 | 26-06-2003 |
| | | | CA | 2432341 A1 | 18-07-2002 |
| | | | CA | 2432350 A1 | 18-07-2002 |
| | | | WO | 02055692 A2 | 18-07-2002 |
| | | | WO | 02055693 A2 | 18-07-2002 |
| | | | EP | 1349927 A2 | 08-10-2003 |
| | | | EP | 1352061 A2 | 15-10-2003 |
| | | | ES | 2204360 T1 | 01-05-2004 |
| | | | US | 2004001811 A1 | 01-01-2004 |
| | | | ZA | 200304500 A | 18-11-2003 |
| | | | WO | 03033700 A1 | 24-04-2003 |
| | | | WO | 03035868 A1 | 01-05-2003 |
| | | | WO | 03035869 A1 | 01-05-2003 |
| | | | WO | 03035870 A1 | 01-05-2003 |
| | | | WO | 03035082 A1 | 01-05-2003 |
| | | | WO | 03035083 A1 | 01-05-2003 |
| | | | WO | 03035876 A1 | 01-05-2003 |
| | | | EP | 1438405 A1 | 21-07-2004 |
| | | | EP | 1438406 A1 | 21-07-2004 |
| | | | EP | 1438409 A1 | 21-07-2004 |
| | | | EP | 1438056 A1 | 21-07-2004 |
| | | | US | 2004038921 A1 | 26-02-2004 |
| | | | US | 2004121348 A1 | 24-06-2004 |
| | | | US | 2004091457 A1 | 13-05-2004 |
| | | | US | 2004126791 A1 | 01-07-2004 |

EPO FORM P0459

For more details about this annex : see Official Journal of the European Patent Office, No. 12/82